# EUROPEAN PATENT SPECIFICATION

(11) **EP 1 962 871 B1**
(45) Date of publication and mention of the grant of the patent: **28.01.2015**
(21) Application number: 06802090.8
(22) Date of filing: 23.08.2006
(51) Int. Cl.: A61K 35/28

(54) **ENHANCED DELIVERY OF CELLS**
VERBESSERTE ABGABE VON ZELLEN
APPORT AMELIORE DE CELLULES

(30) Priority: 16.09.2005 US 227278
(43) Date of publication of application: 03.09.2008
(73) Proprietor: Boston Scientific Scimed, Inc., Maple Grove, MN 55311-1566 (US)
(72) Inventor: FREYMAN, Toby, MA 01453 (US); PALASIS, Maria, MA 02481 (US); NAIMARK, Wendy, MA 02138 (US)
(74) Representative: Peterreins, Frank
(86) International application number: PCT/US2006/032767
(87) International publication number: WO 2007/037850

(56) References cited:
- ASSMUS BIRGIT ET AL: "Transplantation of Progenitor Cells and Regeneration Enhancement in Acute Myocardial Infarction (TOPCARE-AMI)." CIRCULATION 10 DEC 2002, vol. 106, no. 24, 10 December 2002 (2002-12-10), pages 3009-3017, XP002432990 ISSN: 1524-4539 cited in the application
- JAIN PRAVEER ET AL: "Pharmacological management of acute myocardial infarction" CLINICAL CARDIOLOGY, vol. 15, no. 11, 1992, pages 795-803, XP009083603 ISSN: 0160-9289
- KELLY R V ET AL: "Incidence and management of no-reflow following percutaneous coronary interventions", AMERICAN JOURNAL OF MEDICAL SCIENCES, LIPPINCOTT WILLIAMS AND WILKINS, US, vol. 329, no. 2, 1 February 2005 (2005-02-01), pages 78-85, XP009142693, ISSN: 0002-9629
- BACHER A ET AL: "Pentoxifylline attenuates the increase in whole blood viscosity after transfusion", ACTA ANAESTHESIOLOGICA SCANDINAVICA, vol. 49, no. 1, January 2005 (2005-01), pages 41-46, ISSN: 0001-5172

## Description

### FIELD OF THE INVENTION

The present invention relates a pharmaceutical product for use in repairing or replacing heart tissue as defined in the claims.

### BACKGROUND OF THE INVENTION

Congestive heart failure - the ineffective pumping of the heart caused by the loss or dysfunction of heart muscle cells - is a leading cause of death in the United States. A major cause of congestive heart failure is a heart attack, known medically as a myocardial infarction. Standard reperfusion therapies for restoring heart function include surgical revascularization with bypass operation, administration of clot-busting drugs, and/or interventional cardiology such as PTCA (percutaneous transluminal coronary angioplasty), balloon angioplasty, and stent implantation.

Stem cell therapy shows promise as a means to repair and/or replace the cells vital to heart health, particularly the cardiomyocytes which comprise the heart muscle and contract to pump blood, the vascular endothelial cells which form the inner lining of new blood vessels, and smooth muscle cells which form the walls of blood vessels. In vitro studies have shown that stem cells can be induced to develop into new cardiomyocytes and vascular endothelial cells. See Stem Cells: Scientific Progress and Future Research Directions, Chapter 9: Can Stem Cells Repair a Damaged Heart?, Department of Health and Human Services (2001), available at http://stemcells.nih.gov/info/scireport/chapter9.asp. Clinical studies have shown that intracoronary infusion of stem cells may beneficially affect postinfarction remodeling processes. Assmus et al., "Transplantation of Progenitor Cells and Regeneration Enhancement in Acute Myocardial Infarction," Circulation 106:3009 (2002), available at http://circ.ahajournals.org/cgi/content/full/106/24/3009.

Jain Preveer et al., Clin. Cardiology, Vol. 15, No. 11, 1992, pages 795-803 describes drugs used in acute myocardial infarction.

Kelly et al., Am. J. Medical Sciences, Vol. 329, No. 23, February 2005, pages 78-85 describes the use of the vasodilator adenosine in the treatment of the no-reflow-phenomenon occurring during percutaneous coronary intervention. Bacher, et al., Anaesthesiologica Scandinavica, Vol. 49, No.1, January 2005, pages 41-46 describes the administration of the vasodilator pentoxifylline before and during packed red-blood cell transfusion.

Intracoronary infusion allows local delivery of therapeutic formulations to tissue by infusion through a blood vessel. Intracoronary infusion is less invasive than treatments that require opening of the thoracic cage such as some standard reperfusion therapies and intramyocardial transplantation whereby cells are injected directly into the muscle. However, infusion of cells through a blood vessel may cause occlusion of the blood vessel, particularly for the infusion of large cells.

### SUMMARY OF THE INVENTION

The present invention relates to a pharmaceutical product for use in repairing or replacing heart tissue, comprising (i) cells; and (ii) a vasodilator or a vascular permeability enhancer, or both, wherein the cells and the vasodilator and/or the vascular permeability enhancer are to be administered by infusion through a blood vessel and wherein the cells are not of human embryonic origin.

The cells may be stem cells. The vasodilator and/or the vascular permeability enhancer may be administered prior to administering the stem cells. The administration of the stem cells and the administration of the vasodilator and/or the vascular permeability enhancer may be performed by simultaneous intracoronary infusion. One embodiment is the simultaneous administration of mesenchymal stem cells and adenosine II by intracoronary infusion.

In one embodiment, a saline pre-infusate is infused, for example comprising plasma proteins, before the stem cells are to be administered.

### DETAILED DESCRIPTION OF EMBODIMENTS OF THE INVENTION

In one embodiment, stem cells are administered by intracoronary infusion and a vasodilator, a vascular enhancer, or both is/are administered.

The stem cells, the vasodilator, and/or the vascular permeability enhancer may be administered in any order or in multiple doses. Preferably, the vasodilator and/or the vascular permeability enhancer are administered close in time to the administration of the stem cells such that the vasodilator and/or the vascular permeability enhancer improve the delivery of the stem cells to the myocardial tissue. The vasodilator and/or the vascular permeability enhancer may be administered prior to and/or simultaneously with the delivery of the stem cells. The vasodilator and/or the vascular permeability enhancer may be administered by any means known in the art, including, for example, intravenous administration and intracoronary administration. Preferably, the vasodilator and/or the vascular permeability enhancer are administered such they affect only the local environment. Thus, they are preferably administered at the site of stem cell administration. Accordingly, in a preferred embodiment, the stem cells, the vasodilator, and/or the vascular permeability enhancer are all administered by intracoronary infusion.

In one embodiment, the stem cells are administered by intracoronary infusion, which generally encompasses infusing the stem cells into the vascular tree of coronary arteries, arterioles, and capillaries. Preferably, the infusion is performed into the infarct artery. Intracoronary infusion of stem cells is generally performed by using a catheter to deliver the stem cells into the blood vessel. For instance, a balloon catheter is advanced into a previously implanted stent. To allow for adhesion and potential transmigration of the infused cells through the endothelium, the balloon is inflated with low pressure to completely block blood for about 3 minutes while the stem cell suspension is infused distally to the occluding balloon through the central port of the balloon catheter. The method may be repeated more than once. Multiple infusions may be interrupted by short periods of about 3 minutes of reflow by deflating the balloon to minimize extensive ischemia. See Assmus et al., "Transplantation of Progenitor Cells and Regeneration Enhancement in Acute Myocardial Infarction," Circulation 106:3009 (2002), available at http://circ.ahajournals.org/cgi/content/full/106/24/3009. The stem cells may be harvested and prepared for infusion by any means known in the art.

The present invention also contemplates that stem cells may be administered through blood vessels other than the vascular tree of coronary arteries, arterioles, and capillaries. The administration of the stem cells, the vasodilator, and/or the vascular permeability enhancer can be performed by infusion into a blood vessel generally. The blood vessel may lead, for example, to the heart, brain, liver, kidney, pancreas, or lung.

Stem cells include, but are not limited to, non-human embryonic stem cells such as early embryonic stem cells and blastocyst embryonic stem cells; fetal stem cells; umbilical cord stem cells; and adult stem cells such as mesenchymal stem cells, hematopoietic stem cells, endothelial stem cells, peripheral blood stem cells, and multipotent somatic stem cells. In one embodiment, mesenchymal stem cells are preferred.

In order to prevent or reduce the rejection of transplanted cells, the administered stem cells in some embodiments are preferably autologous. The autologous stem cells may be harvested from any source, for example, from bone marrow or peripheral blood.

The present invention may also be useful for delivery of cells for gene therapy. In this embodiment, allogenic rather than autologous cells may be preferred.

A vasodilator enhances the ability of administered stem cells to traverse through the blood vessels and capillaries to the desired site of transplantation, such as the myocardial tissue. Vessel dilation improves delivery of the stem cells and reduces the risk of myocardial ischemia secondary to capillary occlusion. The vasodilator may also reduce the infarct size. Vasodilators useful for the present invention include, but are not limited to, endogenous/metabolic vasodilators such as lactic acid, adenosine triphosphate, adenosine diphosphate, adenosine monophosphate, adenosine, adenosine II, nitric oxide, hemoxygenase, VEGF, and agents causing hypercapnia, hypoxia/hypoxemia, or hyperemia; phosphodiesterase inhibitors such as dipyridamole and sildenafil; sympathetic activity inhibitors such as clonidine and methyldopa; smooth muscle relaxants such as papaverine, hydralazine, dihydralazine, and nitroprusside; beta receptor agonists such as dopamine, dobutamine, arbutamine, albuterol, salmeterol, and isoproterenol; alpha receptor antagonists such as doxazosin, terazosin, and prazosin; organic nitrates, such as glyceryl trinitrate, isosorbide dinitrate, and isosorbide mononitrate; angiotensin converting enzyme (ACE) inhibitors such as benazepril, captopril, enalapril, fosinopril, lisinopril, quinapril, and ramipril; angiotensin II antagonists (or ATI receptor antagonists) such as valsartane, losartan, and candesartan; calcium channel blockers such as amlodipine, nicardipine, nimodipine, felodipine, isradipine, diltiazem, verapamil, and nifedipine; prostaglandins such as alprostadil; and endothelium-dependent vasodilators; and also the vasodilators minoxidil, nitroglycerin, bosentan, eporprostenol, and treprostinil. In one embodiment, the vasodilator is preferably adenosine II, hydralazine, minoxidil, nitroglycerin, an angiotensin converting enzyme inhibitor, bosentan, eporporstenol, treprostinil, or a calcium channel blocker, and most preferably adenosine II.

Because the required vasodilatation may need only to be short lasting, adenosine is a particularly useful vasodilator. Adenosine is an endogenous substance, and it has a very short-lasting action as evidenced by a blood pool half-life of only a few seconds. Vasodilatation will accordingly be most intense at the site of administration, since the drug will tend to reach more distal tissues in less than pharmacologically active concentrations.

A vascular permeability enhancer enhances the ability of the administered stem cells to pass through the vessel wall to the desired site of transplantation, such as the myocardial tissue. Since the cells reach the myocardial tissue via the vascular/capillary bed, agents which enhance vascular permeability are expected to also enhance the levels of stem cells which reach the myocardial tissue. Vascular permeability enhancers useful for the present invention include, but are not limited to, serotonin, bradykinin, platelet-activating factor, prostaglandin E1, histamine, vascular endothelium growth factor, zona occludens toxin, interleukin-2, plasma kinins, L-N-monomethyl arginine, L-N-nitro-arginine methyl ester, alcohol such as ethanol and isopropanol, polyethylene glycols, fatty acid molecules with 10 to 20 carbon rings and certain mono-, di-, and triglycerides of fatty acids.

The delivery of cells to the desired site of transplantation may also be enhanced by administering a saline pre-infusate before administering the stem cells. Infusing saline before infusing the stem cells increases the hydrostatic and/or osmotic pressure, thereby driving the stem cells into the interstitium. A saline pre-infusate that contains plasma proteins may further enhance interstitial transport. The present invention may be useful to repair or replace damaged heart tissue. Without being bound by theory, it is believed that stem cells replace or repair damaged heart tissue by cell-associated myocardial regeneration and neovascularization. Accordingly, preferred subjects of administration for the present invention include subjects, particularly human subjects, suffering from damaged or diseased heart tissue. An especially preferred subject is a human who has suffered an acute myocardial infarction (AMI).

## Claims

1. A pharmaceutical product for use in repairing or replacing heart tissue, comprising:
(i) cells; and
(ii) a vasodilator or a vascular permeability enhancer, or both,
wherein the cells and the vasodilator and/or the vascular permeability enhancer are to be administered by infusion through a blood vessel and wherein the cells are not of human embryonic origin.

2. The pharmaceutical product according to claim 1 for use in repairing or replacing heart tissue, wherein the cells, the vasodilator, and/or the vascular permeability enhancer is/are to be administered in any order or in multiple doses.

3. The pharmaceutical product according to claim 1 or 2 for use in repairing or replacing heart tissue, wherein the vasodilator and/or the vascular permeability enhancer is/are to be administered prior to the cells.

4. The pharmaceutical product according to claim 1 for use in repairing or replacing heart tissue, wherein the vasodilator, and/or the vascular permeability enhanceris/are to be administered simultaneously with the cells.

5. The pharmaceutical product according to claim 1 to 4, wherein the cells are stem cells.

6. The pharmaceutical product according to claim 5 for use in repairing or replacing heart tissue, wherein the stem cells are nonhuman embryonic stem cells, adult stem cells, mesenchymal stem cells, hematopoietic stem cells, endothelial stem cells, peripheral blood stem cells, or multipotent somatic stem cells.

7. The pharmaceutical product according to claim 5 for use in repairing or replacing heart tissue, wherein the stem cells are autologous.

8. The pharmaceutical product use according to claim 1 to 4 for use in repairing or replacing heart tissue, wherein the vasodilator is hydralazine, minoxidil, nitroglycerin, an angiotensin converting enzyme inhibitor, bosentan, eporporstenol, treprostinil, or a calcium channel blocker.

9. The pharmaceutical product according to claim 1 to 4 for use in repairing or replacing heart tissue, wherein the vascular permeability enhancer is serotonin, bradykinin, platelet-activating factor, prostaglandin E₁, histamine, vascular endothelium growth factor, zona occludens toxin, interleukin-2, plasma kinins, L-N-monomethyl arginine, or L-N-nitro-arginine methyl ester.

10. The pharmaceutical product according to claim 1 to 4 for use in repairing or replacing heart tissue, wherein a saline pre-infusate is to be infused before the cells are to be administered.

11. The pharmaceutical product according to claim 10 for use in repairing or replacing heart tissue, wherein the saline pre-infusate comprises plasma proteins.

12. The pharmaceutical product according to claim 1 to 4 for use in repairing or replacing heart tissue, wherein the vasodilator is adenosine, adenosine triphosphate, adenosine diphosphate or adenosine monophosphate.

## Patentansprüche

1. Ein pharmazeutisches Erzeugnis zur Verwendung beim Reparieren oder Ersetzen von Herzgewebe, umfassend:
(i) Zellen; und
(ii) ein Vasodilator oder ein Gefäßpermeabilitätsverstärker, oder beide, wobei die Zellen und der Vasodilator und/oder der Gefäßpermeabilitätsverstärker durch Infusion durch ein Blutgefäß zu verabreichen sind, und
wobei die Zellen nicht humanen embryonalen Ursprungs sind.

2. Das pharmazeutische Erzeugnis nach Anspruch 1 zur Verwendung beim Reparieren oder Ersetzen von Herzgewebe,
wobei die Zellen, der Vasodilator und/oder der Gefäßpermeabilitätsverstärker in jeglicher Reihenfolge oder in mehreren Dosen zu verabreichen ist/sind.

3. Das pharmazeutische Erzeugnis nach Anspruch 1 oder 2 zur Verwendung beim Reparieren oder Ersetzen von Herzgewebe,
wobei der Vasodilator und/oder der Gefäßpermeabilitätsverstärker vor den Zellen zu verabreichen ist/sind.

4. Das pharmazeutische Erzeugnis nach Anspruch 1 zur Verwendung beim Reparieren oder Ersetzen von Herzgewebe,
wobei der Vasodilator und/oder der Gefäßpermeabilitätsverstärker simultan mit den Zellen zu verabreichen ist/sind.

5. Das pharmazeutische Erzeugnis nach Anspruch 1 bis 4,
wobei die Zellen Stammzellen sind.

6. Das pharmazeutische Erzeugnis nach Anspruch 5 zur Verwendung beim Reparieren oder Ersetzen von Herzgewebe,
wobei die Stammzellen nichtmenschlichen embryonale Stammzellen, adulte Stammzellen, mesenchymale Stammzellen, hämatopoietische Stammzellen, endotheliale Stammzellen, periphere Blutstammzellen oder multipotente somatische Stammzellen sind.

7. Das pharmazeutische Erzeugnis nach Anspruch 5 zur Verwendung beim Reparieren oder Ersetzen von Herzgewebe,
wobei die Stammzellen autolog sind.

8. Das pharmazeutische Erzeugnis nach Anspruch 1 bis 4 zur Verwendung beim Reparieren oder Ersetzen von Herzgewebe,
wobei der Vasodilator Hydralazin, Minoxidil, Nitroglycerin, ein Hemmer des Angiotensin-konvertierenden Enzyms, Bosentan, Epoprostenol, Treprostinil oder ein Calciumkanalblocker ist.

9. Das pharmazeutische Erzeugnis nach Anspruch 1 bis 4 zur Verwendung beim Reparieren oder Ersetzen von Herzgewebe,
wobei der Gefäßpermeabilitätsverstärker Serotonin, Bradykinin, plättchenaktivierender Faktor, Prostaglandin E1, Histamin, vaskular endothelialer Wachstumsfaktor, Zona occludens Toxin, Interleukin-2, Plasmakinine, L-N-Monomethyl-Arginine oder L-N-Nitroarginin-Methylester ist.

10. Das pharmazeutische Erzeugnis nach Anspruch 1 bis 4 zur Verwendung beim Reparieren oder Ersetzen von Herzgewebe,
wobei ein Salin-Präinfusat zu infundieren ist bevor die Zellen zu verabreichen sind.

11. Das pharmazeutische Erzeugnis nach Anspruch 10 zur Verwendung beim Reparieren oder Ersetzen von Herzgewebe,
wobei das Salin-Präinfusat Plasmaproteine umfasst.

12. Das pharmazeutische Erzeugnis nach Anspruch 1 bis 4 zur Verwendung beim Reparieren oder Ersetzen von Herzgewebe,
wobei der Vasodilator Adenosin, Adenosintriphosphat, Adenosindiphosphat, oder Adenosinmonophosphat ist.

## Revendications

1. Produit pharmaceutique pour une utilisation dans la réparation ou le remplacement de tissu cardiaque, comprenant :
(i) des cellules ; et
(ii) un vasodilatateur ou un améliorateur de la perméabilité vasculaire, ou les deux ;
dans lequel les cellules et le vasodilatateur et/ou l'améliorateur de la perméabilité vasculaire sont destinés à être administrés par perfusion par l'intermédiaire d'un vaisseau sanguin, et dans lequel les cellules ne sont pas d'origine embryonnaire humaine.

2. Produit pharmaceutique selon la revendication 1, pour une utilisation dans la réparation ou le remplacement de tissu cardiaque, dans lequel les cellules, le vasodilatateur et/ou l'améliorateur de la perméabilité vasculaire sont destinés à être administrés dans un ordre quelconque ou en doses multiples.

3. Produit pharmaceutique selon la revendication 1 ou 2, pour une utilisation dans la réparation ou le remplacement de tissu cardiaque, dans lequel le vasodilatateur et/ou l'améliorateur de la perméabilité vasculaire sont destinés à être administrés préalablement aux cellules.

4. Produit pharmaceutique selon la revendication 1, pour une utilisation dans la réparation ou le remplacement de tissu cardiaque, dans lequel le vasodilatateur et/ou l'améliorateur de la perméabilité vasculaire sont destinés à être administrés simultanément avec les cellules.

5. Produit pharmaceutique selon les revendications 1 à 4, dans lequel les cellules sont des cellules souches.

6. Produit pharmaceutique selon la revendication 5, pour une utilisation dans la réparation ou le remplacement de tissu cardiaque, dans lequel les cellules souches sont des cellules souches embryonnaires non humaines, des cellules souches adultes, des cellules souches mésenchymateuses, des cellules souches hématopoïétiques, des cellules souches endothéliales, des cellules souches du sang périphérique, ou des cellules souches somatiques multipotentes.

7. Produit pharmaceutique selon la revendication 5, pour une utilisation dans la réparation ou le remplacement de tissu cardiaque, dans lequel les cellules souches sont autologues.

8. Produit pharmaceutique selon les revendications 1 à 4, pour une utilisation dans la réparation ou le remplacement de tissu cardiaque, dans lequel le vasodilatateur est l'hydralazine, le minoxidil, la nitroglycérine, un inhibiteur de l'enzyme de conversion de l'angiotensine, le bosentan, l'époprosténol, le tréprostinil, ou un inhibiteur des canaux calciques.

9. Produit pharmaceutique selon les revendications 1 à 4, pour une utilisation dans la réparation ou le remplacement de tissu cardiaque, dans lequel l'améliorateur de la perméabilité vasculaire est la sérotonine, la bradykinine, le facteur d'activation des plaquettes, la prostaglandine E₁, l'histamine, le facteur de croissance de l'endothélium vasculaire, la toxine de *Zonula occludens,* l'interleukine-2, les kinines plasmatiques, la L-N-monométhyl-arginine, ou la L-N-nitro-arginine méthylester.

10. Produit pharmaceutique selon les revendications 1 à 4, pour une utilisation dans la réparation ou le remplacement de tissu cardiaque, dans lequel un préperfusat salin est destiné à être perfusé avant l'administration des cellules.

11. Produit pharmaceutique selon la revendication 10, pour une utilisation dans la réparation ou le remplacement de tissu cardiaque, dans lequel le préperfusat salin comprend des protéines plasmatiques.

12. Produit pharmaceutique selon les revendications 1 à 4, pour une utilisation dans la réparation ou le remplacement de tissu cardiaque, dans lequel le vasodilatateur est l'adénosine, l'adénosine triphosphate, l'adénosine diphosphate ou l'adénosine monophosphate.
